# EUROPEAN PATENT APPLICATION

(11) **EP 2 561 863 A1**
(43) Date of publication of application: **27.02.2013**
(21) Application number: 11178279.3
(22) Date of filing: 22.08.2011
(51) Int. Cl.: A61K 9/19, A61K 31/506, A61K 47/48

(54) **Pharmaceutical compositions comprising voriconazole**

(71) Applicant: FARMAPROJECTS, S.A.U., 08902 L'Hospitalet de Llobregat Barcelona (ES)
(72) Inventor: Bueno Celador, Ignacio, 31014 Pamplona (ES); Bergua Canudo, Virginia, 22002 Huesca (ES); Amela Navarro, Joaquín, 08902 L'Hospitalet de Llobregat (Barcelona) (ES); Toro del Valle, María Isabel, 08902 L'Hospitalet de Llobregat (Barcelona) (ES); Pladevall Rosés, Mireia, 08902 L'Hospitalet de Llobregat (Barcelona) (ES)
(74) Representative: Pons Ariño, Angel

(57) **Abstract**

A solid pharmaceutical composition comprising voriconazole, or a pharmaceutically acceptable salt thereof, at least a cyclodextrin, and at least a sugar.

## Description

### FIELD OF THE INVENTION

The present invention relates to a solid pharmaceutical composition comprising voriconazole, or a pharmaceutically acceptable salt thereof, at least a cyclodextrin, and at least a sugar.

### BACKGROUND OF THE INVENTION

Voriconazole is a triazole antifungal agent disclosed in EP 440 372. In particular it is prepared according to Example 7, as 2R, 3S isomer, by resolution from the enantiomeric pair B of voriconazole. It has the following chemical structure:

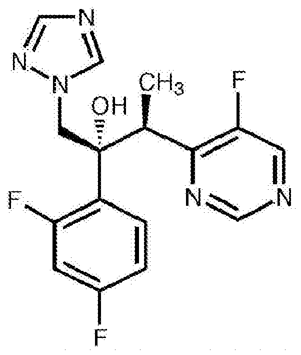

Voriconazole has a low aqueous solubility, and it is not stable in water, because an inactive enantiomer is formed from recombination of the retro-aldol products of hydrolysis, thus, development of pharmaceutical compositions is difficult. For example, developing an aqueous intravenous composition with a sufficient shelf life is difficult, due to the fact that the compound has to be in contact with water prior to the administration to the patent. On the other hand, the complexity in the development of oral composition would be due if wet granulation is pretended to be used to obtain a granulate.

EP 440 372 describes that the compounds therein disclosed can be administered orally and they can be injected parenterally. In that document it is only mentioned that compound in an aqueous medium may be improved by complexation with a hydroxyalkyl derivative of a cyclodextrin. However, the present inventors have discovered that the composition comprising only a cyclodextrin, as disclosed in the EP 440 372, does not show good stability. So, voriconazole is not stable in all the cyclodextrins suitable for pharmaceutical use of the market. Degradation impurities have found in some compositions with some cyclodextrins. These impurities are hydrolysis and oxidation products of voriconazole.

In the WO 91/11172 discloses sulphoalkylether cyclodextrin derivatives of formula (A) 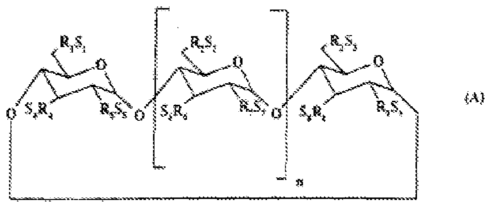 wherein
n is 4, 5 or 6;
R₁₋₉ independently represent O- or O-(C₂₋₆ alkylene-SO-, provided that at least one of R₁ and R₂ is O-(C₂₋₆ alkylene)-SO-; and
S₁₋₉ independently represents a pharmaceutically acceptable cation (such as H+ or Na+).

The WO 98/058677 discloses pharmaceutical compositions comprising voriconazole, or a pharmaceutically acceptable derivative thereof, and a cyclodextrin derivative of formula I, 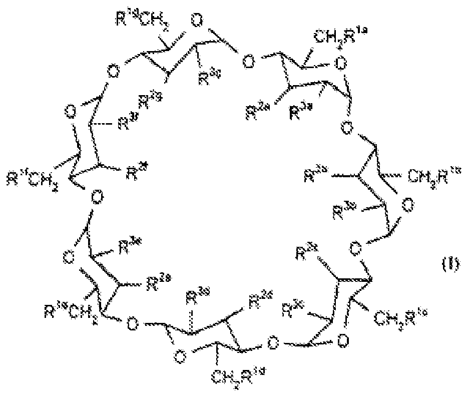 wherein
R^{1a-g}, R^{2a-g} and R^{3a-g} independently represent OH or O(CH₂)₄SO₃H;
provided that at least one of R^{1a-g} represents O(CH₂)₄SO₃H;
or a pharmaceutically acceptable salt thereof.

It is stated that the solubility of voriconazole in water can be increased by molecular encapsulation with sulphoalkylether cyclodextrin derivatives of the type disclosed in International Patent Application WO 91/11172 and wherein the cyclodextrin ring is substituted by at least one sulphobutyl group. In addition, these cyclodextrin derivatives would enable the finished lyophilised product to accommodate high levels of moisture (up to 3.0%) without a detrimental effect on stability, and also controls and minimises the formation of the inactive enantiomer of voriconazole.

The WO 2007047253 discloses complexes and mixtures of antifungal azole compound in a complex or mixture with a hydroxybutenyl cyclodextrin may increase the bioavailability of an antifungal azole compound, allowing for smaller doses to achieve therapeutic responses comparable to larger doses of the antifungal azole compound alone. However, WO 2007047253 is silent about how to increase the stability of such compositions.

The EP 2018866 and EP 2027850 describe a process for improving the solubility of voriconazole in aqueous solutions with β-cyclodextrin. The process comprises (i) preparing an aqueous solution of voriconazole and a beta-cyclodextrin; (ii) keeping the solution obtained in step (i) comprising voriconazole and a beta-cyclodextrin at a temperature of at least 40°C to obtain a composition comprising voriconazole and a beta-cyclodextrin.

The WO 2011020605 describes a process for producing a co-evaporate comprising (a) voriconazole, (b) cyclodextrin and (c) optionally an organic solvent, said process comprising the steps of (i) dissolving voriconazole and cyclodextrin in the organic solvent, and (ii) removing the solvent completely or partially. Furthermore, it is disclosed the use of said co-evaporate for the production of a voriconazole-cyclodextrin complex. The aim of the process is to provide an oral dosage form having superior dissolution properties.

Other approaches have been disclosed to improve the stability of pharmaceutical composition comprising voriconazole. For example, WO 97/28169 discloses a phosphate pro-drug of voriconazole, which exhibits increased solubility and aqueous stability. However, the pro-drug does not exhibit 100% bioequivalence to voriconazole, such that cost of goods is again significantly increased.

### SUMMARY OF THE INVENTION

The present invention relates to pharmaceutical composition comprising voriconazole with an improved solubility which confers a sufficient shelf life to the composition. The pharmaceutical composition as herein disclosed are stable and, for example, the formation of degradation impurities is reduced, and specially the formation of the inactive enantiomer of voriconazole. The good stability of the pharmaceutical composition entails that they can be used when the use of water is mandatory, as for example in case of parenteral administration, as the voriconazole solution is stable even after been reconstituted. Moreover, the pharmaceutical composition are easy to obtain, specially if water is necessary during the manufacturing process, e.g. lyophilization, since both the voriconazole solution during the lyophilization process and the final freeze dried composition are stable. The advantages of the pharmaceutical composition are not limited to the stability, but they also improve the low solubility of voriconazole in water. In addition, the present compositions are very versatile which entails that it can comprise well-known and long-time used cyclodextrins with no toxicity problems.

Various aspects of the present invention are described below

According to the first aspect of the present invention there is provided a stable solid, preferably a powder for solution for infusion, pharmaceutical composition comprising voriconazole, or a pharmaceutically acceptable salt thereof, at least a cyclodextrin, and at least a sugar. The inventors have surprisingly found that the addition of a sugar to the complex voriconazole:hydroxypropyl-β-cyclodextrins stabilizes the composition avoiding the formation of hydrolysis and oxidation products.

According to the second aspect of the present invention there is provided a process for preparing a stable pharmaceutical composition as herein disclosed, comprising at least the following steps of:
a) adding voriconazole to an aqueous solution of a cyclodextrin; and
b) mixing the solution obtained in step a) with the sugar; and
c) lyophilising the composition obtained in step b).

According to the third aspect of the present invention there is provided a pharmaceutical composition according for use as a medicament, particularly for treating a fungal infection.

### DEFINITIONS

The term "stable pharmaceutical composition" as used herein refers to a pharmaceutical composition which when packed in vials and stored at 40±2° C, 75±5% relative humidity for a period of 3 months, the amount of total impurities are less than 2.5 % w/w, preferably below 1.0 %, and more preferably below 0.6 %. Further, the term "stable" as used herein for lyophilized forms refers to a pharmaceutical composition which when reconstituted in a sterile liquid vehicle, the reconstituted solutions are clear without precipitation of the water insoluble drug for at least 2 hours after addition of the sterile liquid vehicle.

The term "cyclodextrin" includes alpha.-cyclodextrin, beta.-cyclodextrin and gamma.-cyclodextrin. The termcyclodextrin also include a pharmaceutically acceptable derivative of cyclodextrins suitable for pharmaceutical use. For example, such derivative of cyclodextrins are those in which at least one atom selected from hydrogen, oxygen or carbon in the cyclodextrin molecule is replaced by an atom or a group of atoms ordinarily present as a substituent in this kind of organic compounds (saccharides). These derivatives include etherified cyclodextrins, branched cyclodextrins, acylated cyclodextrins and sulfur-containing cyclodextrins. Said etherified cyclodextrins include alkylcyclodextrins such as methylcyclodextrin, ethylcyclodextrin, propylcyclodextrin, dimethylcyclodextrin, trimethylcyclodextrin etc., alkenylcyclodextrins, hydroxyalkylcyclodextrinssuch as hydroxyethylcyclodextrin, hydroxypropylcyclodextrin etc., alkoxyalkylcyclodextrins, aralkylcyclodextrins such as benzylcyclodextrin etc., haloalkylcyclodextrins such as chloroethylcyclodextrin etc., andcylodextrinepichlorohydrine copolymer and so on. These may be etherified cyclodextrins in which one, two or three hydroxy groups in any of the glucose units of the cyclodextrin molecule are converted into ether. Said branched cyclodextrins include glucosylcyclodextrin, maltosylcyclodextrin etc. Said acylated cyclodextrins include alkanoylcyclodextrins such as formylcyclodextin, acetylcyclodextrin etc. Said sulfur-containing cyclodextrins include sulfonated cyclodextrins etc. The derivatives of cyclodextrin include also derivatives in which two or more of derivatizations selected from etherification, branching, acylation and sulfuration are co-existing. These derivatives are known or can be prepared by a method similar to that for the known derivatives. Specific example of cyclodextrins are carboxyethyl α-cyclodextrin, carboxyethyl β-cyclodextrin, carboxyethyl γ -cyclodextrin, carboxymethyl α-cyclodextrin, carboxymethyl α-cyclodextrin, carboxymethyl α-cyclodextrin, carboxymethyl α-cyclodextrin, carboxymethyl α-cyclodextrin, carboxymethyl β-cyclodextrin, carboxymethyl β-cyclodextrin, ethyl γ-cyclodextrin, γ-cyclodextrin acetate, γ-cyclodextrin acetate, hexakis-(2,6-di-O-methyl-3-0-n-pentyl)- α-cyclodextrin, hydroxyethyl β-cyclodextrin, hydroxyethyl β-cyclodextrin and hydroxypropyl-β-cyclodextrin.

The term "alkyl cyclodextrins" refers to all forms of alkyl cyclodextrins, including alkyl-α, β or γ cyclodextrins. The term alkyl cyclodextrin having one or more neutral substituents selected from the group consisting of straight chained or branched C1-10 alkyl, preferably C1-4 alkyl selected from methyl, ethyl, propyl and butyl, where each substituent is substituted with one or more hydroxyl groups. In a specific aspect, the cyclodextrin has at least one neutral substituent selected from the group consisting of 2-hydroxypropyl or 3-hydroxypropyl substituents. Examples of alkyl cyclodextrins are hydroxypropyl-γ-cyclodextrin, dimethyl-β-cyclodextrin and hydroxypropyl-β-cyclodextrin.

A cryoprotectant is a substance that protects from freezing damage.

The term "oral administration" refers to the provision of the pharmaceutical composition via the mouth through ingestion, or via some other part of the gastrointestinal system including the esophagus. Examples of oral dosage forms include tablets (including compressed, coated or uncoated), capsules, hard or soft gelatin capsules, pellets, pills, powders, granules, elixirs, tinctures, colloidal dispersions, dispersions, effervescent compositions, films, sterile solutions or suspensions, syrups and emulsions and the like.

As used herein, the term "parenteral administration" refers to and includes any route through which a compound is administered to a mammal other than through the digestive tract, non limiting examples of such routes include: intravenous injection, intra-arterial injection, intracavitary injection, intramuscular injection, and injection through an intravenous line, cannula, catheter, or the like.

As used herein the term "ophthalmic composition" refers to a composition intended for application to the eye or its related or surrounding tissues such as, for example, eyelid. The term also includes compositions intended to therapeutically treat conditions of the eye itself or the tissues surrounding the eye and compositions administered via the ophthalmic route to treat therapeutically a local condition other than that involving the eye. The ophthalmic composition can be applied topically or by other techniques, known to persons skilled in the art, such as injection to the eye or its related tissues. Examples of suitable topical administration to the eye include administration in eye drops and by spray pharmaceutical compositions. A further suitable topical administration route is by subconjunctival injection.

The term "sugar" refers to any type of simple carbohydrate, such as a mono or disaccharide, or a combination thereof, either naturally obtained, refined from a natural source, or artificially produced. A variety of sugars are known and used in various pharmaceutical compositions, including without limitation, sucrose, glucose, mannose, fructose, lactose, etc. As such, the recitation of the term "sugar" generically, should be interpreted to include such specific compounds, as well as others not expressly recited.

The term "lyophilization" refers to a drying process by freezing a material dissolved in water below 0º C and then reducing the surrounding pressure using a vacuum pump to allow the frozen water in the material to sublimate.

The term "powder for solution for infusion" as used herein consists of a powder, such as lyophilisate that can be quickly reconstituted (extemporaneously) in solution with water to be administered by parenteral route. The powder is typically reconstituted with 19 ml of water for injections to obtain an extractable volume of 20 ml of clear concentrate containing 10 mg/ml of voriconazole. For administration, the required volume of the reconstituted concentrate is added to a recommended compatible infusion to obtain a final voriconazole solution normally containing a voriconazole concentration of 0.5-5 mg/ml.

### DETAILED DESCRIPTION OF THE INVENTION

According to the first aspect of the present invention there is provided a solid pharmaceutical composition comprising voriconazole, or a pharmaceutically acceptable salt thereof, at least a cyclodextrin, and at least a sugar. Preferred solid pharmaceutical composition is a powder for solution for infusion.

The sugar may be selected from a monosaccharide, a disaccharide and mixtures thereof. Examples of those sugars are glucose (dextrose), fructose (levulose), galactose, xylose, ribose, fructose, lactose, maltose, trehalose, cellobiose, and mixtures thereof. Preferred sugars are lactose, trehalose, and mixtures thereof. In a preferred embodiment the sugar is lactose.

The cyclodextrin may be selected from the group of cyclodextrin acetates, carboxyalkylated cyclodextrins, cyclodextrin phosphates, cyclodextrin succinates, cyclodextrin sulfates, alkyl cyclodextrins, hydroxyalkyl cyclodextrins and mixtures thereof, preferably the cyclodextrin is a alkyl cyclodextrins, hydroxyalkyl cyclodextrins and mixtures thereof, and more preferably a hydroxyalkyl cyclodextrin. In a particular embodiment the cyclodextrin is an α-cyclodextrin, a β-cyclodextrin, a γ-cyclodextrin or mixtures thereof. It is of particular interest that the pharmaceutical composition comprises a complex voriconazole:cyclodextrin.

Good results have been obtained when the pharmaceutical composition as disclosed herein comprises alkyl cyclodextrins as hydroxyalkyl-β-cyclodextrin. The solubility of voriconazole with an alkyl cyclodextrin can be improved by complexation with an alkyl cyclodextrins, particularly when a hydroxypropyl-β-cyclodextrin is used.

In a particular embodiment, the pharmaceutical compositions as disclosed herein are adapted for parenteral, oral or ophthalmic administration. More particularly, the pharmaceutical composition as herein disclosed is suitable for parenteral administration, preferably for intravenous administration. The most preferred pharmaceutical composition is a powder for solution for infusion, preferably comprising between 9.0 mg/mL and 11.0 mg/mL of voriconazole after reconstitution. Preferably, the powder for solution for infusion is a vial comprising a single dose sterile lyophile.

In another particular embodiment, the molar ratio voriconazole:cyclodextrin is from 10:1 to 1:15. Preferably, the molar ratio voriconazole:cyclodextrin ranges from 1:2 to 1:8. In a most preferred embodiment the molar ratio voriconazole:cyclodextrin is from 1:3 to 1:6.

The amount of sugar, or the total amount of sugars, ranges normally from 5 to 50 % by weight. Preferably, it ranges from 10 to 35 % by weight. More preferably, it ranges from 17 to 28 % by weight.

The amount of voriconazole typically ranges from 1 to 15 % by weight. Preferably, it ranges from 2 to 8 % by weight.

In another particular embodiment, the stable pharmaceutical composition as disclosed herein, consists essentially of: voriconazole, or a pharmaceutically acceptable salt thereof; a cyclodextrin or mixtures of cyclodextrins; and a sugar or mixtures of sugars. In preferred embodiment, the stable pharmaceutical composition as disclosed herein consists of: voriconazole, or a pharmaceutically acceptable salt thereof; a cyclodextrin or mixtures of cyclodextrins; and a sugar or mixtures of sugars. In an even more preferred embodiment, the stable pharmaceutical composition as disclosed herein, consists of: voriconazole, a cyclodextrin, and a sugar.

In case that the pharmaceutical composition as disclosed herein are a lyophilized composition, the pH is preferably from 3 to 8 before the composition is lyophilized, more preferably the pH is between 4 and 5.

In a particular embodiment there is provided a stable pharmaceutical composition comprising voriconazole, or a pharmaceutically acceptable salt thereof, at least a cyclodextrin, and at least a sugar, wherein: the pharmaceutical composition is a powder for solution for infusion; the sugar is selected from a monosaccharide, a disaccharide and mixtures thereof; the cyclodextrin is selected from the group of cyclodextrin acetates, carboxyalkylated cyclodextrins, cyclodextrin phosphates, cyclodextrin succinates, cyclodextrin sulfates, alkyl cyclodextrins, hydroxyalkyl cyclodextrins and mixtures thereof; the molar ratio voriconazole:cyclodextrin is from 10:1 to 1:15; the amount of sugar, or the total amount of sugars, ranges from 5 to 50 % by weight; and the amount of voriconazole ranges from 1 to 15 % by weight.

In another particular embodiment there is provided a stable pharmaceutical composition comprising voriconazole, or a pharmaceutically acceptable salt thereof, at least a cyclodextrin, and at least a sugar, wherein: the pharmaceutical composition is a powder for solution for infusion; the sugar is selected from glucose (dextrose), fructose (levulose), galactose, xylose, ribose, fructose, lactose, maltose, trehalose, cellobiose, and mixtures thereof; the cyclodextrin is a alkyl cyclodextrins, hydroxyalkyl cyclodextrins and mixtures thereof; the molar ratio voriconazole:cyclodextrin ranges from 1:2 to 1:8; the total amount of sugars, ranges from 5 to 50 % by weight; the amount of voriconazole ranges from 1 to 15 % by weight.

In another particular embodiment there is provided a stable pharmaceutical composition comprising voriconazole, or a pharmaceutically acceptable salt thereof, at least a cyclodextrin, and at least a sugar, wherein: the pharmaceutical composition is a powder for solution for infusion; the sugar is lactose, trehalose, and mixtures thereof; the cyclodextrin a hydroxyalkyl-β-cyclodextrin; the molar ratio voriconazole:cyclodextrin ranges from 1:2 to 1:8; the amount of sugar, or the total amount of sugars, ranges from 5 to 50 % by weight; and the amount of voriconazole ranges from 1 to 15 % by weight.

According to the second aspect of the present invention there is provided a process for preparing a stable pharmaceutical composition as herein disclosed, comprising at least the following steps of:
a) adding voriconazole to an aqueous solution of a cyclodextrin; and
b) mixing the solution obtained in step a) with the sugar; and
c) lyophilising the composition obtained in step b).

Preferably, in step (a) the mixture is stirred until the formation of a clear solution. This fact would imply that the complex voriconazole cyclodextrin has been formed. It is also advisable to stir the mixture formed in step (b) until obtaining complete dissolution. Optionally, the process comprises a filtration after the step (b) but before lyophilising. The lyophilization is preferably carried out in the vial wherein the composition will be later reconstituted with water. Once the lyophilization is carried out, the vial may be vacuum closed a nd encapsulated. In a particular embodiment there is provided a pharmaceutical composition obtainable by any one of the process disclosed in the second aspect.

The following examples illustrate the pharmaceutical composition as disclosed herein and an a method for obtaining thereof and are not intended to limit the scope of the invention set forth in the claims appended thereto.

### EXAMPLES

### Method of preparation

The following procedure was used for the manufacture of the below three examples.

### Step 1: Dissolution

The cyclodextrin was weighted and added to 2/3 of the total volume of water for injections. The total volume of water is the amount of water necessary to have a final concentration of voriconazole of 10.0 mg/mL before the lyophilization.

The mixture was stirred with helix until complete dissolution (motor at 900 rpm). N₂ was bubbled in the surface of the solution and also inside. The content of O₂ was measured and it was closed to 0% before adding voriconazole.

Voriconazole was weighted and added to the cyclodextrin solution. The mixture was stirred with helix until complete dissolution (motor at 1500 rpm). The manufacturing process was carried out in inert atmosphere.The sugar was weighted and added it to the solution (only for examples 1 and 2), and the final mixture was stirred with helix until complete dissolution (motor at 900 rpm).

It was added the remaining water for injections up to have a concentration of voriconazole 10.0 mg/ML. The pH was measured.

### Step 2: Filtration

Vacuum filtration through 0.2 µm polyethersulfone filter. It was repeated twice.
Control the appearance of the solution. It was clear, colourless and without any visible particles.
It was measured the density of the solution.

### Step 3: Dosage

It was dosed according to the density or its equivalent volume. It was pre-close the vial with stopper special for lyophilization process.

### Step 4: Freeze drying process

The vials were freeze dried, vacuum closed and encapsulated.

Three pharmaceutical compositions were manufactured following the method of preparation described in this section. The cyclodextrin used is the commercial HPBCD (Kleptose®).

### Example 1:

i.v. pharmaceutical composition of voriconazole with hydroxypropyl-β-cyclodextrin and lactose:

| COMPONENT | QUANTITY (mg per ml of solution before lyophilization) | QUANTITY |
|---|---|---|
| Voriconazole | 10,0 | 5.000,0 |
| Hydroxypropyl-β-cyclodextrin | 160,0 | 80.000,0 |
| Lactose monohydrate | 55,0 | 27.500,0 |

### Example 2:

i.v. pharmaceutical composition of voriconazole with hydroxypropyl-β-cyclodextrin and trehalose:

| COMPONENT | QUANTITY (mg per ml of solution before lyophilization) | QUANTITY |
|---|---|---|
| Voriconazole | 10,0 | 5.000,0 |
| Hydroxypropyl-β-cyclodextrin | 160,0 | 80.000,0 |
| Trehalose | 55,0 | 27.500,0 |

### Example 3:

i.v. pharmaceutical composition of voriconazole with hydroxypropyl-β-cyclodextrin with no sugar:

| COMPONENT | QUANTITY (mg per ml of solution before lyophilization) | QUANTITY |
|---|---|---|
| Voriconazole | 10,0 | 5.000,0 |
| Hydroxypropyl-β-cyclodextrin | 160,0 | 80.000,0 |

### Pre-stability Tests results:

After manufacturing, they were stored at 40°C and 75% HR. And the quantity of impurities generated was measured at time zero, in 1 month, in 2 months and in 3 months.

The impurities considered as main products of degradation for voriconazole in the final dosage form are Impurity A and Impurity C, which according to the European Pharmacopoeia 7.3 (page 4026-4028) are:
Impurity A: 1-(2,4-difluorophenyl)-2-(1H-1,2,4-triazol-1-yl)ethanone .
Impurity C: 4-ethyl-5 fluoropyrimidine.

These degradation products were determined by HPLC analysis and are described in the following table in percentage of each impurity regarding the total amount of drug product: and the total is the percentage of impurities taking into account all the impurities present in the drug product.

| | | **Example 1** | **Example 2** | **Example 3** |
|---|---|---|---|---|
| | Time (Months) | lactose | trehalose | No sugar |
| Impurity A | 0 | 0,003 | 0,003 | 0,021 |
| | 1 | 0,057 | 0,084 | 0,400 |
| | 2 | 0,105 | 0,121 | 0,590 |
| | 3 | 0,142 | 0,148 | 1,137 |
| Impurity C | 0 | 0,002 | 0,001 | 0,017 |
| | 1 | 0,060 | 0,061 | 2,577 |
| | 2 | 0,107 | 0,118 | 0,598 |
| | 3 | 0,151 | 0,178 | 1,153 |
| TOTAL | 0 | 0,084 | 0,085 | 0,103 |
| | 1 | 0,206 | 0,250 | 5,806 |
| | 2 | 0,311 | 0,415 | 1,434 |
| | 3 | 0,477 | 0,503 | 2,785 |

## Claims

1. A solid pharmaceutical composition comprising voriconazole, or a pharmaceutically acceptable salt thereof, at least a cyclodextrin, and at least a sugar.

2. The pharmaceutical composition according to claim 1, wherein the pharmaceutical composition is a powder for solution for infusion.

3. The pharmaceutical composition according any one of the preceding claims, wherein the sugar is selected from a monosaccharide, a disaccharide and mixtures thereof.

4. The pharmaceutical composition according to the preceding claim, wherein the sugar is lactose, trehalose, and mixtures thereof, preferably the sugar is lactose.

5. The pharmaceutical composition according to any one of the preceding claims, wherein the cyclodextrin is selected from the group of cyclodextrin acetates, carboxyalkylated cyclodextrins, cyclodextrin phosphates, cyclodextrin succinates, cyclodextrin sulfates, alkyl cyclodextrins hydroxyalkyl cyclodextrins and mixtures thereof.

6. The pharmaceutical composition according to any one of the preceding claims, wherein the cyclodextrin is selected from the group of alkyl cyclodextrins.

7. The pharmaceutical composition according to any one of the preceding claims, wherein the cyclodextrin is hydroxyalkyl-β-cyclodextrin, preferably the cyclodextrin is hydroxypropyl-β-cyclodextrin.

8. The pharmaceutical composition according to any one of the preceding claims, for parenteral, oral or ophthalmic administration.

9. The pharmaceutical composition according to any one of the preceding claims, for parenteral administration, preferably for intravenous administration.

10. The pharmaceutical composition according to any one of the preceding claims, wherein the molar ratio of voriconazole:cyclodextrin from 1:2 to 1:8, preferably is from 1:3 to 1:6.

11. The pharmaceutical composition according to any one of the preceding claims, wherein the amount of sugar, or the total amount of sugars, ranges from 5 to 50 % by weight.

12. The pharmaceutical composition according to any one of the preceding claims, wherein the amount of voriconazole ranges from 1 to 15 % by weight.

13. The pharmaceutical composition according to any one of the preceding claims, wherein the pharmaceutical composition is a lyophilized composition wherein the pH is from 3 to 8 before the composition is lyophilized, preferably the pH is between 4 and 5.

14. A process for preparing a pharmaceutical composition as defined in any one of the claims 1 to 13, comprising at least the following steps of:
a) adding voriconazole to an aqueous solution of a cyclodextrin; and
b) mixing the solution obtained in step a) with the sugar; and
c) lyophilising the composition obtained in step b).

15. The pharmaceutical composition according to any one of the claims 1 to 13, for treating a fungal infection.
